# EUROPEAN PATENT APPLICATION

(11) **EP 1 864 635 A1**
(43) Date of publication of application: **12.12.2007**
(21) Application number: 06729861.2
(22) Date of filing: 24.03.2006
(51) Int. Cl.: A61F 13/49, A61F 5/44, A61F 13/511, A61F 13/53

(54) **ABSORBENT ARTICLE**

(30) Priority: 29.03.2005 JP 2005094493
(71) Applicant: UNICHARM CORPORATION, Ehime 799-0111 (JP)
(72) Inventor: YAGO, Toshiya c/o Technical Center, Uni-Charm Co., Kagawa, 7691602 (JP)
(74) Representative: Eke, Philippa Dianne
(86) International application number: PCT/JP2006/305913
(87) International publication number: WO 2006/104024

(57) **Abstract**

It is intended to provide an absorbent article which is appropriately usable as a urine pad and by which urine can be surely trapped in both of small urine leakage and relatively large urine leakage. A liquid-permeable sheet (20) is provided on the surface of an absorbent (13) which is made of pulp and a highly water-absorbing resin. This liquid-permeable sheet (20) together with a skin-contacting part (21) having an elastic member (25) and a side fixing part (22) from a rise (23). The rise (23) has a deflection part (23a) formed therein. On the absorbent, a cavity surrounded by the skin-contacting part and the rise is formed. When urine is supplied in a relatively large amount, the urine is pooled in the cavity (26) and then absorbed by the absorbent (13).

## Description

### TECHNICAL FIELD

This application is based on and claims the benefit of priority from Japanese Patent Application No. 2005-94493, the content of which is incorporated herein for reference.

The present invention relates to an absorbent article having liquid absorbing functions and, in particular, to an absorbent article suitable for use as a urine collection pad.

### BACKGROUND ART

Absorbent articles used as urine collection pads include liquid-permeable surface sheets, liquid-impermeable back sheets, and absorbent bodies positioned between a surface sheet and a back sheet. The absorbent bodies are generally formed by mixing pulp and a highly water-absorbent resin. The pulp is superior in its function of instantaneously absorbing liquids, and the highly water-absorbent resin has the ability to absorb and hold large amounts of liquid.

Mild incontinence occurs when there is abdominal pressure such as by sneezing. The amount of urine excreted is small with respect to such mild incontinence. The excreted urine does not rush through the urethra, but easily moves along the skin. The urine moving along the skin cannot be consistently transferred into the absorbent body unless the absorbent article and an excretory part of the body can adhere to each other, so that the urine easily leaks out to the absorbent article. Furthermore, the urine is in contact with the skin for a long time, which easily damages the skin.

Thus, an absorbent article used as a urine collection pad must be able to absorb, when supplied large amounts of urine, the urine quickly and reliably, and are required to reliably collect small amounts of urine excreted. This is true for absorbent articles used as sanitary napkins.

As described in Japanese Unexamined Patent Application, Publication No. 9-313529 and Japanese Unexamined Patent Application, Publication No. 2002-320638, examples of conventional absorbent articles include an absorbent article having a raised ridge portion extending in a longitudinal direction formed on a surface thereof on the liquid receiving side. The absorbent article easily collects a small amount of liquid excreted from an excretory part in such a manner that the raised ridge portion can adhere to the excretory part.

When the raised ridge portion is formed of a liquid-absorbent sheet, as disclosed in Japanese Unexamined Patent Application, Publication No. 9-313529, however, urine or the like remains in the raised ridge portion becoming wet, so that a portion of the skin that comes into contact with the raised ridge portion easily becomes wet. The skin in a wet state and the absorbent article rub against each other, which easily causes irritation of the skin. When the amount of liquid in the raised ridge portion, for example, when a lot of urine is excreted, liquid permeation meets with resistance from the raised ridge portion. This causes a large amount of liquid to be temporarily stored in both side parts of the raised ridge portion. When a large amount of liquid is excreted, pulp cannot absorb the liquid fast enough, so that the liquid easily flows outward on both right and left sides of the absorbent article.

### DISCLOSURE OF THE INVENTION

### Problems to be Solved by the Invention

The present invention has been made in order to solve the abovementioned conventional problems and has an objective thereof to provide an absorbent article that can absorb, when a small amount of liquid is supplied thereto, the liquid, and makes it difficult for the liquid to remain in contact with the skin, and easily collects, when a large amount of liquid is supplied thereto, the liquid without leaking outward.

### Means for Solving the Problems

In an aspect of the present invention an absorbent article having an absorbent body, a liquid-permeable sheet provided on the liquid receiving side of the absorbent body, and a back sheet provided on the side opposite to the wearing side of the absorbent body and in a vertically-long shape such that a size thereof in a longitudinal direction is greater than a size thereof in a transverse direction, in which the liquid-permeable sheet has a skin contacting portion extending along a center line in the longitudinal direction, side fixing portions respectively fixed to a surface on the liquid receiving side of the absorbent body on both sides of the skin contacting portion, and a pair of raised portions connecting the skin contacting portion and the side fixing portions, the skin contacting portion being provided with at least one elastic expansion/contraction portion, and spaced apart from the surface of the absorbent body with an elastic contractive force in the longitudinal direction of the at least one elastic expansion/contraction portion, and at least one cavity surrounded by the skin contacting portion and the raised portions and extending in the longitudinal direction is formed on the absorbent body.

For example, the at least one elastic expansion/contraction portion includes a plurality of elastic expansion/contraction portions spaced apart in the transverse direction, and the skin contacting portion is spaced a predetermined width apart from the surface of the absorbent body. Furthermore, an elastic member extending in the longitudinal direction may be joined to the liquid-permeable sheet, to form the at least one elastic expansion/contraction portion.

In the absorbent article according to the present invention, the skin contacting portion easily adheres to an excretory part of the human body. When a small amount of urine or the like is excreted, the urine can be collected in the skin contacting portion, and the urine or the like can be transferred into the absorbent body after moving along the liquid-permeable sheet. On the other hand, when a large amount of urine or the like is supplied, the urine is absorbed by the absorbent body after passing through the liquid-permeable sheet. However, the urine or the like that cannot be absorbed is absorbed by the absorbent body after being temporarily stored within the cavity. Therefore, even when a large amount of urine or the like is supplied, side leakage does not easily occur.

In the present invention, it is preferable that the liquid-permeable sheet forming the raised portions has deflection allowance between the side fixing portions and the skin contacting portion, respectively.

When the raised portion has deflection allowance, the internal capacity of the cavity can be increased. Therefore, a large amount of urine or the like can be temporarily stored within the cavity, so that the urine or the like does not easily leak.

In the present invention, it is preferable that the skin contacting portion has a first region and a second region positioned behind the first region, the height thereof raised from the surface of the absorbent body in the first region is greater than that in the second region, and the at least one cavity includes cavities formed in both the first region and the second region, respectively.

In the absorbent article according to the present invention, the first region comes into contact with the urine excretory part, and the second region comes into contact with the vicinity of the anus to the vicinity of the buttocks. In the first region, the skin contacting portion is spaced greatly apart from the absorbent body. Therefore, the first region easily adheres to the urine excretory part, and the second region does not readily impart an uncomfortable feeling in a crotch portion of the body because the skin contacting portion is low. Moreover, the cavity is formed in not only the first region, but also the second region. Therefore, when a large amount of urine is excreted at one time, the urine can be temporarily stored in the cavity in a long range ahead of and behind the absorbent article, which readily prevents side leakage.

For example, the elastic expansion/contraction portion is provided in the first region, while not being provided in the second region.

The distance in the width direction between a base end of one of the pair of raised portions and a base end of the other raised portion in the first region may be greater than that in the second region. In such a configuration, the cross-sectional area of the cavity in the first region can be made greater than that of the cavity in the second region.

In the present invention, it is preferable for the absorbent body to have leakage preventing walls each formed of a side sheet and a second elastic member, and extending in the longitudinal direction in both side parts on its surface on the liquid receiving side, and that the leakage preventing walls be curved in a state where the surface on the liquid receiving side of the absorbent body is recessed with an elastic contractive force of the second elastic member.

When the absorbent body is concavely curved with the absorbent body worn on the crotch portion and the leakage preventing walls are raised in both of the side parts, it is possible to hold, when a large amount of urine is supplied, the urine between the concavely-curved absorbent body and the leakage preventing walls on both sides. Therefore, the urine that cannot be absorbed by the absorbent body does not easily leak from the side parts.

In the present invention, it is preferable that the absorbent body has absorbing core compression portions formed by compressing an absorbing core in the absorbent body, the absorbing core compression portions being spaced right and left apart from the center line in the longitudinal direction and formed continuously in the longitudinal direction with a predetermined width.

When the compression portions are positioned in both side parts of the skin contacting portion, a liquid can be quickly absorbed by the high-density compression portions. Moreover, both side ends of the absorbent body are not stiff because no compression portion is provided at the side ends. Therefore, a worn feeling obtained when the absorbent body is brought into contact with the crotch portion is improved.

There can be provided a liquid-permeable surface sheet covering both the skin contacting portion and the side fixing portions. For example, the surface sheet is fixed to the surface of the skin contacting portion.

When the skin contacting portion is covered with the surface sheet, the skin contacting portion is not directly visible. Therefore, the absorbent article is easy to employ, for example, by elderly persons, because the appearance thereof on the liquid receiving side is improved.

It is preferable that the surface sheet has strechability. The stretchability can be indicated by subjecting the surface sheet to mesh embossing. Alternatively, the stretchability can be indicated by forming the surface sheet of a material having elastic stretchability. The stretchability of the surface sheet makes it easy to space the skin contacting portion apart from the absorbent body, and allows the degree of freedom of the skin contacting portion to be increased.

The surface sheet may have through portions such as slits extending in the longitudinal direction formed in both of the side parts of the skin contacting portion.

When the through portions are formed, the skin contacting portion is easily raised from the surface of the absorbent body without being bound by the surface sheet. When a large amount of urine is temporarily supplied, the urine can be directly transferred into the absorbent body after passing through the through portions. Therefore, the absorbent body can quickly absorb the large amount of urine.

### Effects of the Invention

The absorbent article according to the present invention easily adheres to the excretory part of the human body and easily collects a small amount of excretory fluid. When the urine or the like is supplied in large amounts, the urine or the like does not easily leak outward, and can be quickly and reliably absorbed by the absorbent body.

### BRIEF DESCRIPTION OF THE DRAWINGS

Fig. 1 is a perspective view showing an absorbent article according to a first embodiment of the present invention;
Fig. 2 is a plan view showing a state where the absorbent article according to the first embodiment is unfolded in a planar shape;
Fig. 3 is a cross-sectional view taken along a line III-III shown in Fig. 2;
Fig. 4 is a cross-sectional view taken along a line IV-IV shown in Fig. 2;
Fig. 5 is a cross-sectional view showing an absorbent article according to a second embodiment of the present invention;
Fig. 6 is a plan view showing a state where an absorbent article according to a third embodiment of the present invention is unfolded in a planar shape; and
Fig. 7 is a cross-sectional view taken along a line VII-VII shown in Fig. 6.

### PREFERRED MODE FOR CARRYING OUT THE INVENTION

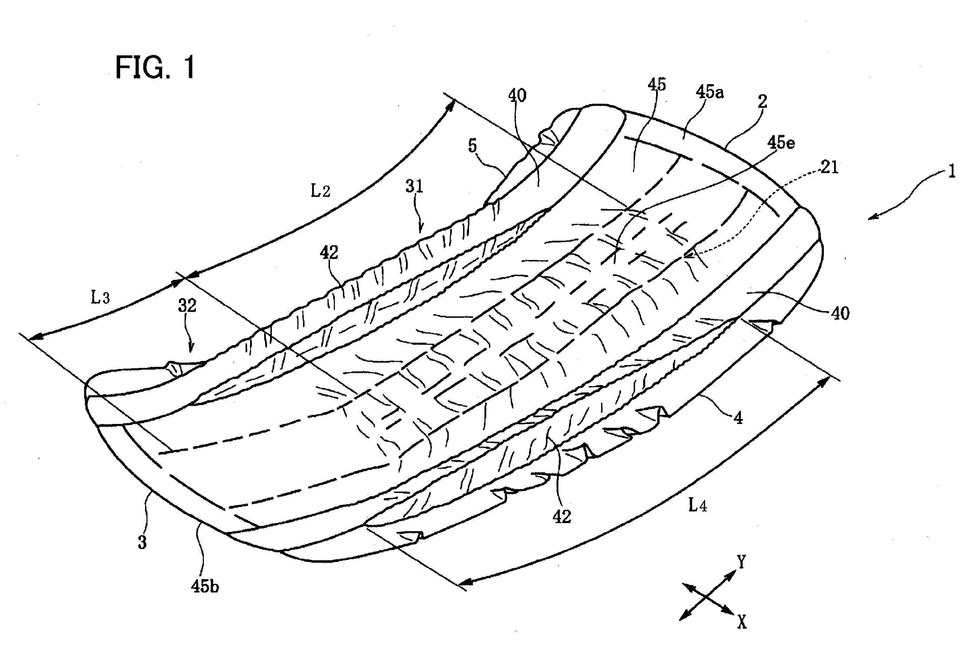

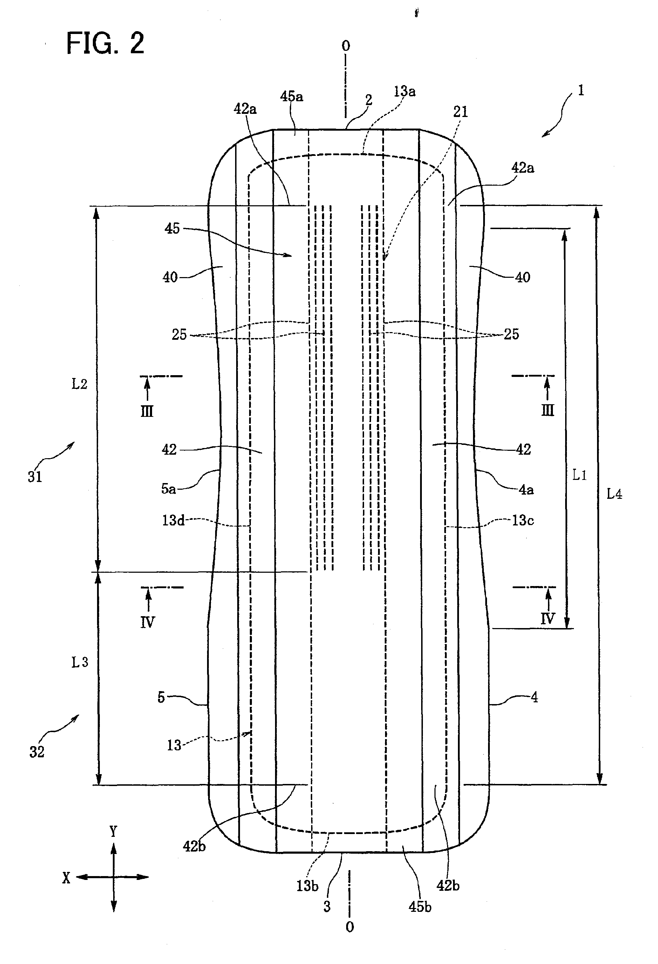

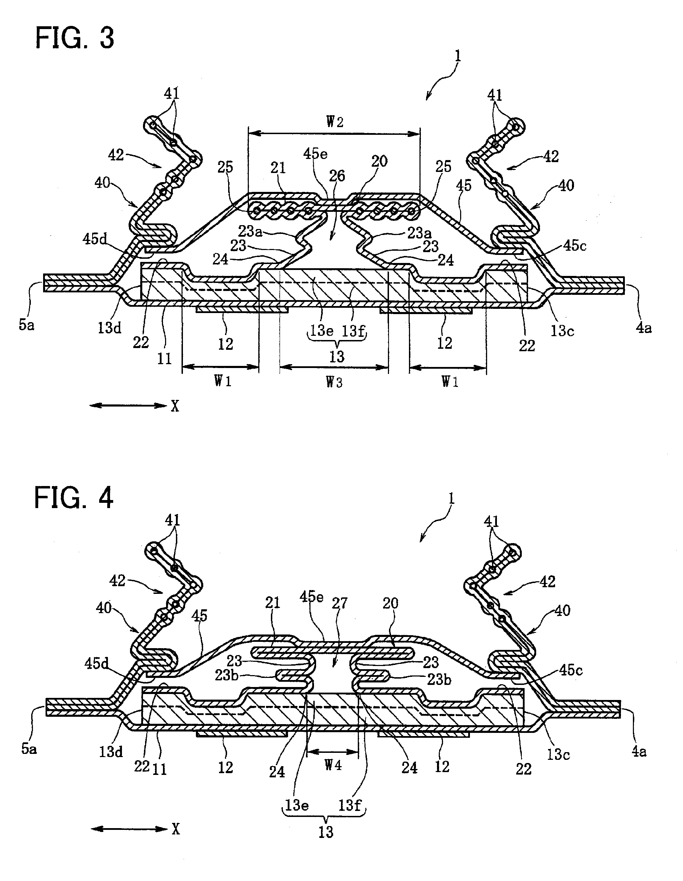

Fig. 1 is a perspective view showing an absorbent article 1 according to a first embodiment of the present invention; Fig. 2 is a plan view showing a state where the absorbent article is unfolded in a planar shape from its surface on the liquid receiving side; Fig. 3 is a cross-sectional view taken along a line III-III of the absorbent article 1 shown in Fig. 2; and Fig. 4 is a cross-sectional view taken along a line IV-IV.

The absorbent article 1 is suitable for use by an elderly female incontinent patient, easily collects urine excreted in small amounts due to stress unitary incontinence, and can exhibit a function of collecting and absorbing the urine, when a relatively large amount of urine is temporarily excreted due to urge incontinence. The absorbent article 1 has a vertically-long shape in which a size thereof in a longitudinal direction (a Y direction) is greater than the size thereof in a width direction (an X direction) that is a transverse direction. The size in the longitudinal direction is approximately 300 to 500 mm, and the size in the width direction is approximately 100 to 200 mm. Furthermore, the thickness of the absorbent article 1 is approximately 5 to 30 mm in a state where it is worn between underwear and the body.

The absorbent article 1 has a front edge 2, a rear edge 3, a right edge 4, and a left edge 5. As shown in Fig. 2, in a portion with a length L1 in the longitudinal direction, a curved recess 4a directed toward a center line O in the longitudinal direction is formed at the right edge 4, and a recess 5a is also formed at the left edge 5. The absorbent article 1 is worn such that a region having the recesses 4a and 5a formed therein is positioned between the thighs. In the absorbent article 1, the distance from a rear end of the region having the recesses 4a and 5a formed therein to the rear edge 3 is equal to or greater than the distance from a front end of the region to the front edge 2.

As shown in Figs. 3 and 4, a back sheet 11 is provided on the side opposite to the clothing side of the absorbent article 1. The outer shape of the back sheet 11 is the same as the outer shape of the absorbent article 1 shown in Fig. 2, and the edges of the back sheet 11 coincide with the front and rear edges 2 and 3 and the right and left edges 4 and 5, respectively. The back sheet 11 is formed of a liquid-impermeable and air-impermeable resin film such as a polyethylene resin film. Furthermore, the back sheet 11 may be formed of a liquid-impermeable and air-permeable resin film.

Pressure sensitive adhesive layers 12 are provided on an outer surface of the back sheet 11. The pressure sensitive adhesive layer 12 is formed by coating with a hot melt adhesive such as a rubber-based hot melt adhesive. A mold release sheet is bonded to a surface of the pressure sensitive adhesive layer 12, to protect the pressure sensitive adhesive layer 12, which is not illustrated in Figs. 3 and 4, until the absorbent article 1 is employed.

An absorbent body 13 is located on the back sheet 11, and the back sheet 11 and the absorbent body 13 are bonded and fixed to each other with hot melt adhesives or the like. The absorbent body 13 is a mixture of pulp fibers and highly water-absorbent resin. Although the content of the highly water-absorbent resin is approximately 20 to 60% by mass, and the total basis weight of the pulp fibers and the high water-absorbent resin is approximately 500 to 2000 g/m², they are not limited to these numerical values. The absorbent body 13 is formed by laminating an upper layer absorbent body 13e that is a mixture of pulp fibers and highly water-absorbent resin on a lower layer absorbent body 13f, which is also a mixture of pulp fibers and highly water-absorbent resin, and wrapping a laminate in hydrophilic tissue paper.

As shown in Fig. 2, the planar shape of the absorbent body 13 is a rectangular shape having a front edge 13a, a rear edge 13b, a right edge 13c, and a left edge 13d. The front edge 13a and the rear edge 13b of the absorbent body 13 are positioned inside the front edge 2 and the rear edge 3 of the absorbent article 1, and the right edge 13c and the left edge 13d of the absorbent body 13 are positioned inside the right edge 4 and the left edge 5 of the absorbent article 1.

As shown in Figs. 3 and 4, the absorbent body 13 has absorbing core compression portions with a predetermined width W1 formed by compressing an absorbing core in the absorbent body 13 formed at positions thereof equally spaced right and left apart from the center line O in the longitudinal direction. The absorbing core compression portions with the predetermined width W1 extend in a strip shape in the longitudinal direction on both right and left sides, respectively, and the absorbing core compression portions with the predetermined width W1 are continuously formed from the front edge 13a of the absorbent body 13 to the rear edge 13b thereof. Alternatively, a front end of the absorbing core compression portion with the predetermined width W1 may be positioned slightly inside the front edge 13a of the absorbent body 13, and a rear end of the absorbing core compression portion with the predetermined width W1 may be positioned slightly ahead of the rear edge 13b of the absorbent body 13.

The absorbing core compression portions with the predetermined width W1 are formed by embossing the absorbent body 13 to pressurize and compress the embossed absorbent body 13. This embossing causes the absorbent body 13 to be pressurized and compressed such that each of the absorbing core compression portions with the predetermined width W1 has a pattern in the shape of dots arranged in large numbers, or a pattern in a stripe shape or a mesh shape. In the absorbing core compression portion with the width direction W1, the density of the absorbent body 13 is more than that at the center along the center line O in the longitudinal direction, or the thickness thereof is less than that at the center. The absorbing core compression portions with the predetermined width W1 can quickly absorb a liquid because they have a high fiber density. Moreover, the absorbing core compression portions with the predetermined width W1 are not formed in the right edge 13c and the left edge 13d of the absorbent body 13, respectively, so that the right edge 13c and the left edge 13d are not stiff. Therefore, when the absorbent body 13 is worn on a crotch portion of the body, the absorbing core compression portions with the predetermined width W1 make it difficult to impart an uncomfortable feeling to the body. The distance from the right edge 13c or the left edge 13d of the absorbent body 13 to the absorbing core compression portions with the predetermined width W1 is preferably not more than 5 mm, and more preferably not less than 10 mm. Furthermore, the predetermined width W1 of the absorbing core compression portions is approximately 3 to 15 mm.

A liquid-permeable sheet 20 is provided on a surface on the liquid receiving side of the absorbent body 13. The liquid-permeable sheet 20 is hydrophilic and has the function of being permeated by a liquid. Used as the liquid-permeable sheet 20 is preferably that formed of only synthetic resin fibers that have been subjected to hydrophilic treatment and without natural fibers such as pulp fibers having a liquid holding function, highly water-absorbent resin, and water-absorbent resin such as polyvinyl alcohol (PVA). An air-through nonwoven fabric having a basis weight in a range of 10 to 40 g/m² is preferably used as the liquid-permeable sheet 20 such that a superior liquid permeability can be exhibited. The air-through nonwoven fabric is formed of core-sheath conjugate synthetic fibers using polypropylene resin (PP) or polyethylene terephthalate resin (PET) as a core and using polyethylene resin (PE) as a sheath, or PET resin fibers, PP resin fibers, or the like.

However, the liquid-permeable sheet 20 can also include the abovementioned pulp fibers, rayon fibers, or the like.

The liquid-permeable sheet 20 has a constant width in the X direction, which is the transverse direction, and extends continuously in the longitudinal direction (Y direction), the front end and rear end thereof coinciding with the front edge 2 and the rear edge 3 of the absorbent article 1, respectively.

As shown in Figs. 3 and 4, the liquid-permeable sheet 20 forms a skin contacting portion 21, side fixing portions 22 fixed to a surface of the absorbent body 13 on both right and left sides of the skin contacting portion 21, and raised portions 23 connecting the skin contacting portion 21 to the side fixing portions 22, respectively. A boundary line between the side fixing portion 22 and the raised portion 23 is a base end 24 of the raised ridge portion 23. The skin contacting portion 21 and the raised portions 23 on both sides thereof constitute a raised ridge portion. Although the base end 24 is positioned inside the absorbing core compression portion with the predetermined width W1 in Figs. 3 and 4, the base end 24 may be positioned above the absorbing core compression portion with the predetermined width W1.

Although in the present embodiment, the side fixing portion 22 is made to directly adhere to the surface of the absorbent body 13, another hydrophilic sheet or the like may be interposed between the surface of the absorbent body 13 and the side fixing portion 22.

As shown in Fig. 2, the raised ridge portion including the skin contacting portion 21 and the raised portions 23 on both sides thereof is formed over a first region 31 and a second region 32 behind the first region 31. The length L2 of the first region 31 is approximately 130 to 230 mm, and the length L3 of the second region 32 is set to a length that is less than the length L2 of the first region 31. The lengths L2 and L3 may be the same, or the length L3 of the second region 32 may be greater than the length L2 of the first region 31. Fig. 3 is a cross-sectional view of the first region 31, and Fig. 4 is a cross-sectional view of the second region 32.

In the first region 31, the liquid-permeable sheet 20 forming the skin contacting portion 21 is folded in two on both sides with the center line O in the longitudinal direction interposed therebetween. On each of the sides, at least one elastic member 25 is sandwiched between folded portions of the liquid-permeable sheet 20, and a folded inner surface of the liquid-permeable sheet 20 and the elastic member 25 are bonded and fixed to each other with hot melt adhesives. The at least one elastic member 25 includes four elastic members provided on one side and four elastic members on the other side, respectively, with the center line O in the longitudinal direction interposed therebetween. The elastic members 25 are fixed to the liquid-permeable sheet 20 in a state where they are extended by 1.2 to 2 times in the longitudinal direction. In the first region 31, the elastic members 25 form an elastic expansion/contraction portion. In the elastic expansion/contraction portion, an elastic contractive force in the longitudinal direction is exerted on the skin contacting portion 21. The width W2 of the skin contacting portion 21 is approximately 25 to 40 mm.

As shown in Fig. 3, in the first region 31, the liquid-permeable sheet 20 is not stretched in a vertical direction in each of the pair of raised portions 23 raised toward the skin contacting portion 21 from the base ends 24, but has deflection allowing portions 23a provided in the raised portions 23, respectively. The plurality of elastic members 25 extending in the longitudinal direction is made to adhere and be fixed to the deflection allowing portion 23a so that the deflection allowing portion 23a is folded up into concertinas in its portion having the elastic members, when viewed as a cross section shown in Fig. 3.

As shown in Fig. 4, in the second region 32, the liquid-permeable sheet 20 forming the skin contacting portion 21 is also folded in two on both sides with the center line O in the longitudinal direction interposed therebetween, and folded portions of the liquid-permeable sheet 20 are made to adhere to each other on each of the sides. However, in the second region 32, no elastic member 25 is provided in the skin contacting portion 21. In the second region 32, the liquid permeable sheet 20 has folded portions 23b respectively formed in a pair of raised portions 23 extending to the skin contacting portion 21 from base ends 24. In the folded portion 23b, the folded portions of the liquid-permeable sheet 20 are fused and joined to each other. The fusion is heat sealing, ultrasonic sealing, high-frequency sealing, or the like. Alternatively, in the folded portion 23b, the folded portions of the liquid-permeable sheet 20 may be fixed and joined to each other.

As shown in Figs. 3 and 4, the size W3 in the width direction of spacing between the base ends 24, each serving as the boundary line between the side fixing portion 22 and the raised portion 23 in the first region 31, is made greater than the size W4 in the width direction of spacing between the base ends 24 in the second region 34. The size W3 is not less than 1.2 times, and preferably not less than 1.5 times the size W4. In the second region 32, the folded portions 23b are formed in the raised portions 23, respectively, and the size W4 in the width direction of the spacing between the base ends 24 is made small. Therefore, in the first region 31, the skin contacting portion 21 is greatly spaced apart from the surface of the absorbent body 13 by an elastic contractive force of the elastic members 25. The maximum height raised from the surface on the clothing side of the absorbent article 1, i.e., the surface of the back sheet 11 to the surface on the liquid receiving side of the skin contacting portion 21 is approximately 10 to 45 mm, and preferably not less than 15 mm.

In the first region 31, a cavity 26 is formed within a surface of the absorbent body 13 positioned between the base ends 24, the raised portions 23 having the deflection allowing portions 23a, respectively, and the skin contacting portion 21.

As shown in Fig. 4, the height raised from the surface of the absorbent body 13 to the surface of the skin contacting portion 21 in the second region 32 is less than that in the first region 31. However, the surface of the absorbent body 13 positioned between the base ends 24 and the skin contacting portion 21 positioned thereon do not directly adhere to each other, and a cavity 27 through which a liquid can pass, although the cross-sectional area thereof is small, is formed within the surface of the absorbent body 13 positioned between the base ends 24, the raised portions 23, and the skin contacting portion 21.

It should be noted that a structure between the front end of the first region 31 and the front edge 2 of the absorbent article 1 is the same as that shown in Fig. 4, or the skin contacting portion 21 is made to adhere and be fixed to the side fixing portions 22 positioned therebelow. The same is true for a region between the rear end of the second region 32 and the rear edge 3.

Side sheets 40 each folded in two are provided on both right and left sides, respectively, on the surface on the liquid receiving side of the absorbent article 1. The side sheet 40 is a non-woven fabric formed of hydrophobic fibers. Examples of the non-woven fabric include a point-bonded nonwoven fabric, a spunbonded non-woven fabric, and a meltblown non-woven fabric that are formed of core-sheath conjugate fibers using PP resin as a core and PE resin as a sheath. A liquid-impermeable non-woven fabric is preferably used.

The side sheet 40 positioned on the right side in the figure has an outer edge thereof coinciding with the right edge 4 of the absorbent article 1. The side sheet 40 and the back sheet 11 are bonded and joined to each other with hot melt adhesive outside the right edge 13c of the absorbent body 13. The side sheet 40 and the back sheet 11 are thermally sealed to each other in a range with a predetermined width inside the right edge 13c. The side sheet 40 positioned on the left side in the figure has an outer edge thereof coinciding with the left edge 5 of the absorbent article 1. The side sheet 40 and the back sheet 11 are bonded and joined to each other with a hot melt adhesive outside the left edge 13d of the absorbent body 13. The side sheet 40 and the back sheet 11 are thermally sealed to each other in a range with a predetermined width inside the left edge 13d.

In each of the side sheets 40, a plurality of (five to eight) elastic members 41 extending in the longitudinal direction is fixed to a folded inner surface thereof. The elastic member 41 extends over substantially the entire length in the longitudinal direction of the absorbent article 1, and is made to adhere to the side sheet 40 in a state where it is extended by approximately 1.2 to 1.8 times. In Fig. 2, the length of a range in which the side sheet 40 is spaced apart from the absorbent body 13 to form a leakage preventing wall 42 is indicated by L4. The side sheet 40 is joined to the back sheet 11 in a folded state in a region ahead of the range with the length L4, while being joined to the back sheet 11 in a folded state in a region behind the range with the length L4.

In the range with the length L4, an elastic contractive force of the elastic members 41 causes an elastic contractive force for bringing the front end and the rear end of the absorbent article 1 into contact with each other to be exerted. Accordingly, the absorbent body 13 is deformed in a concavely curved shape such that a surface thereof on the liquid receiving side is recessed, and the side sheet 40 is raised from the surface on the liquid receiving side of the absorbent body 13 to form the leakage preventing wall 42 in the range with the length L4, as shown in Fig. 1. The absorbent body 13 is embossed to be compressed in its ranges with the width W1 on both right and left sides to form absorbing core compression portions with the predetermined width W1, and is relatively high in rigidity. Accordingly, the absorbent body 13 is easily deformed in a concavely curved shape when receiving the elastic contractive force of the elastic members 41. Therefore, a phenomenon in which the absorbent body 13 is wrinkled to be compressed in the longitudinal direction does not easily appear.

As shown in Fig. 2, the leakage preventing wall 42 is formed in a range including both the first region 31 and the second region 32. That is, a front end 42a of the leakage preventing wall 42 is at the same position as or slightly behind the front end of the first region 31, while a rear end 42b of the leakage preventing wall 42 is at the same position as or slightly ahead of the rear end of the second region 32. Furthermore, a range in which the elastic contractive force of the elastic members 41 provided in the side sheet 40 is exerted is the range with length L4. This range is sufficiently longer than the range with length L2 of the first region 31 in which the elastic members 25 provided in the liquid-permeable sheet 20 exhibit the elastic contractive force.

The absorbent body 13 is deformed in a concavely curved shape in the range with length L4. The elastic contractive force is exerted on the skin contacting portion 21 in only the first region 31. Therefore, in the first region 31, the size raised from the surface of the absorbent body 13 to a free end of the leakage preventing wall 42 is greater than the size in which the skin contacting portion 21 is raised from the surface of the absorbent body 13. The same is true for the second region 32.

The surface on the liquid receiving side of the liquid-permeable sheet 20 is covered with a surface sheet 45. As shown in Fig. 2, a front end 45a of the surface sheet 45 is joined to the back sheet 11 with an adhesive or by heat sealing at the front edge 2 of the absorbent article 1, while a rear end 45b of the surface sheet 45 is joined to the back sheet 11 with an adhesive or by heat sealing at the rear edge 3. As shown in Figs. 3 and 4, a right end 45c of the surface sheet 45 is joined to the side sheet 40 with an adhesive or by heat sealing, and a left end 45d of the surface sheet 45 is also joined to the side sheet 40.

Furthermore, the surface sheet 45 is made to adhere to a surface on the liquid receiving side of the skin contacting portion 21 along the center line in the longitudinal direction in both the first region 31 and the second region 32.

The surface sheet 45 is hydrophilic and has the function of being permeated by a liquid. Preferably used as the surface sheet 45 are ones formed of only synthetic resin fibers that have been subjected to hydrophilic treatment and include no natural fibers such as pulp having a liquid holding function, highly water-absorbent resin, and water-absorbent resin such as polyvinyl alcohol (PVA), similarly to the liquid-permeable sheet 20. A through-air nonwoven fabric having a basis weight in a range of 10 to 40 g/m² is preferably used as the surface sheet 45 such that a superior liquid permeating function can be exhibited. The through-air nonwoven fabric is formed of core-sheath conjugate synthetic fibers using polypropylene resin (PP) or polyethylene terephthalate resin (PET) as a core, and polyethylene resin as a sheath, or PET fibers, PP fibers, or the like. Alternatively, the surface sheet 45 may be formed of a resin film having a large number of liquid permeation holes.

Used as the surface sheet 45 is one irregularly diluted to contract in at least one of the longitudinal direction and the transverse direction by mesh embossing. The mesh embossing is performed in the process of meshing a nonwoven fabric or a resin film between a pair of rolls, each having irregularities formed on a peripheral surface thereof. The surface sheet 45 has stretchability in at least one of the longitudinal direction and the transverse direction by passing through the process. As a result, the skin contacting portion 21 covered with the surface sheet 45 is easily raised from the surface of the absorbent body 13, and the skin contacting portion 21, together with the surface sheet 45, moves relatively freely rightward and leftward. It should be noted that the surface sheet 45 may be formed of a nonwoven fabric including elastically stretchable fibers such as polyurethane fibers so that the surface sheet 45 has elastic stretchability.

The absorbent article 1 is mainly suited to be worn by a woman who is an incontinent patient. A mold release sheet provided on the surface on the clothing side of the absorbent article 1 is stripped, to fix the back sheet 11 to an inner surface of underwear through the pressure sensitive adhesive layer 12. The absorbent article 1 enters a concavely curved shape in which a surface thereof on the liquid receiving side is recessed, as shown in Fig. 1, with the elastic contractive forces of the elastic members 41 attached to the side sheets 40 and the elastic members 25 attached to the liquid-permeable sheet 20 in its free state. At the time of wearing, the absorbent article 1 is worn such that the skin contacting portion 21 positioned in the first region 31 is brought into contact with the urine excretory part of the body, and the second region 32 covers the anus to the vicinity of the coccyx.

In the absorbent article 1, the skin contacting portion 21 spaced apart from the absorbent body 13 is covered with the surface sheet 45, and the raised shape of the skin contacting portion 21 and the shape of the compression portions with the width W1 formed in the absorbent body 13 are not visually observed directly. Therefore, it seems to a wearer as if the surface of the absorbent article 1 were smooth. When an elderly woman employs the absorbent article 1, she does not readily feel awkward and uncomfortable about wearing the absorbent article 1 of this type.

In a worn state, the skin contacting portion 21 is spaced apart from the surface of the absorbent body 13 in the first region 31, so that the skin contacting portion 21 easily adheres to the urine excretory part. Furthermore, the skin contacting portion 21 has a degree of freedom for the absorbent body 13 in the first region 31. Therefore, even if the absorbent body 13, together with the underwear, moves, a state where the skin contacting portion 21 adheres to the urine excretory part is easily maintained.

In mild incontinence, for example, as in the case of sneezing, which is referred to as stress unitary incontinence, the amount of urine excreted from the urine excretory part is small. The urine attempts to move along a surface of the body. However, since the skin contacting portion 21 adheres to a region from the urine excretory part to the lowermost part of the crotch portion with the surface sheet 45 sandwiched therebetween, the urine is easily collected by the surface sheet 45 and the skin contacting portion 21. The urine is easily absorbed by the absorbent body 13 after moving along the liquid-impermeable sheet 20.

When a relatively large amount of urine is excreted because a person cannot hold his or her urine until going to a bathroom, for example, which is referred to as urge incontinence, the urine permeates the surface sheet 45, and permeates the liquid-permeable sheet 20, to be absorbed by the absorbent body 13. The absorbent body 13 has pulp and highly water-absorbent resin. Therefore, the urine is quickly absorbed by the pulp, and the large amount of urine is absorbed by the highly water-absorbent resin, and held therein.

There is a case where a large amount of urine is supplied and the pulp cannot absorb the urine fast enough so that the urine remains on the surface of the absorbent body 13. In the absorbent article 1, the absorbent body 13 maintains its concavely curved shape. Therefore, the urine does not easily flow back and forth. Moreover, as shown in Fig. 3, in the first region 31, the cavity 26 interposed between the skin contacting portion 21 and the raised portions 23 exists on the surface of the absorbent body 13. Therefore, the urine is temporarily stored in the cavity 26. Since the surface of the absorbent body 13 is exposed to the inside of the cavity 26, the urine in the cavity 26 is absorbed by the pulp in the absorbent body 13 within a short time. Furthermore, as shown in Fig. 4, in the second region 32, there is also a distance, as indicated by the width W4, between the base ends 24 of the raised portions 23, the liquid-permeable sheet 20 forming the skin contacting portion 21 is opposed thereto without adhering thereto, and the cavity 27 having a small cross-sectional area is formed between the surface of the absorbent body 13 and the skin contacting portion 21. Accordingly, a relatively large amount of urine remains on the surface of the absorbent body 13. Even if the urine is transferred into the second region 32, the urine moves through the cavity 27 to be quickly absorbed by the absorbent body 13 thereunder.

As shown in Fig. 3, in the first region 31, the liquid-permeable sheet 20 forming the raised portions 23 is provided with the deflection allowing portions 23a, to ensure that the inner cross-sectional area of the cavity 26 is quite great. Therefore, when a large amount of urine is supplied to the cavity 26 after passing through the surface sheet 45 and the skin contacting portion 21, the large amount of urine can be stored in the cavity 26. The urine is blocked by the raised portions 23, so as not to easily flow out in the width direction.

Furthermore, when a particularly large amount of urine is excreted, the urine is stored on the surface of the absorbent body 13 that maintains the concavely curved shape thereof, and the leakage preventing walls 42 are present in both its side parts. Therefore, the leakage preventing walls 42 can prevent the movement in the width direction of the urine, which can prevent side leakage.

Thus, the absorbent article 1 easily prevents, when a small amount of urine is excreted at the time of stress unitary incontinence, the urine from moving along the crotch portion of the body, and can also reliably absorb the urine, when a large amount of urine is excreted at the time of urge incontinence. Furthermore, the skin contacting portion 21 and the surface sheet 45 have a low liquid holding function because they are formed of synthetic resin fibers that have been subjected to hydrophilic treatment. Therefore, a state where the skin contacting portion 21 and the surface sheet 45 are wet with urine does not remain for a long time, so that the skin can be prevented from remaining wet. Therefore, problems such as irritation of the crotch portion do not easily occur.

Although another embodiment of the present invention is described, portions other than portions particularly described in the following embodiment are the same as those in the first embodiment. Therefore, the same portions are assigned the same reference numerals and hence, the detailed description is not repeated.

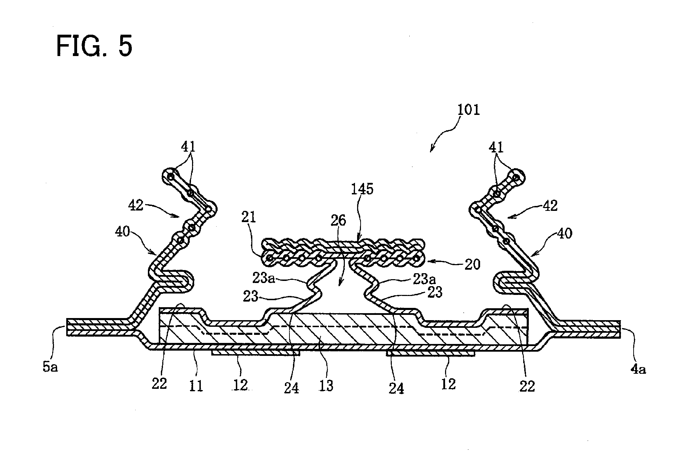

Fig. 5 illustrates an absorbent article 101 according to a second embodiment of the present invention, and is a transverse sectional view in a first region 31, as in Fig. 3.

In the absorbent article 101, a surface sheet 145 is provided only on a surface on the liquid receiving side of a skin contacting portion 21. The surface sheet 145 is formed of the same material as that forming the surface sheet 45 in the first embodiment. The surface sheet 145 is in a strip shape folded in three and extending over the whole length in a longitudinal direction. A front end and a rear end of the surface sheet 145 are joined to a back sheet 11 in the vicinity of a front edge 2 of the absorbent article 1 and the vicinity of a rear edge 3 thereof. Furthermore, the surface sheet 145 is made to adhere and be fixed to the surface of the skin contacting portion 21.

In the absorbent article 101, the skin contacting portion 21 does not experience resistance from the surface sheet 145, so that it can be raised from a surface of an absorbent body 13. Furthermore, the skin contacting portion 21 has a high degree of freedom, so that a state where the skin contacting portion 21 adheres to a urine excretory part is easily maintained.

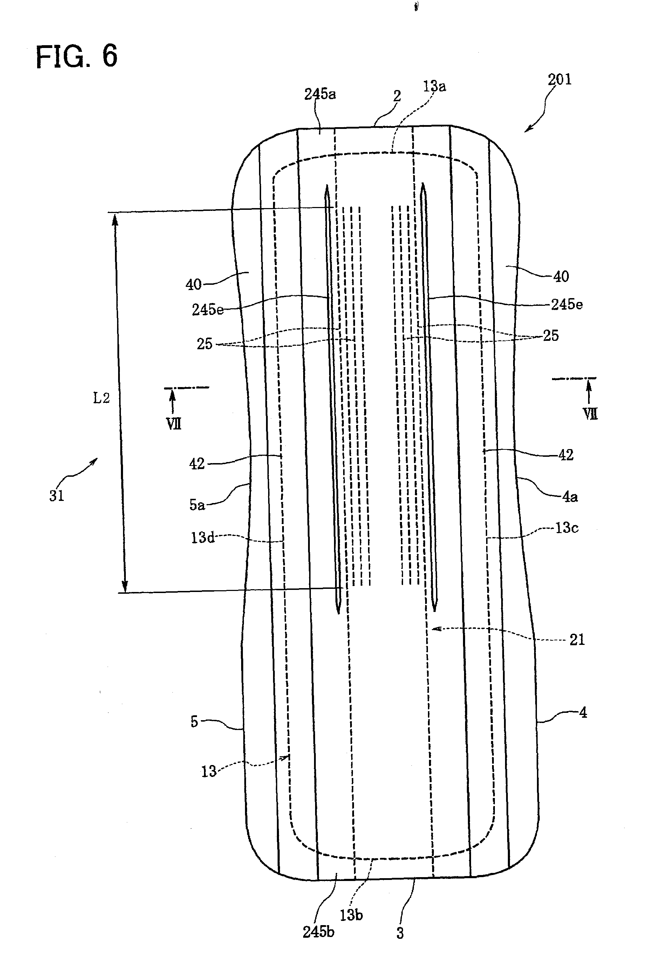

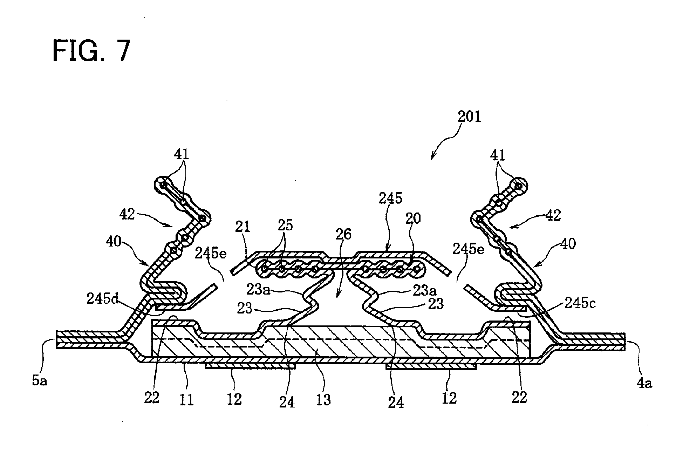

Fig. 6 is a plan view showing an absorbent article 201 according to a third embodiment of the present invention from the liquid receiving side, and Fig. 7 is a cross-sectional view taken along a line VII - VII shown in Fig. 6.

A surface sheet 245 provided in the absorbent article 201 is formed of the same material as that forming the surface sheet 45 in the first embodiment, its front end 245a being joined to a back sheet 11 at a front edge 2 and a rear end 245b thereof being joined to the back sheet 11 at a rear edge 3. Furthermore, a right end 245c is also joined to a side sheet 40, and a left end 245d is also joined to the side sheet 40. A skin contacting portion 21 is covered with the surface sheet 245.

The surface sheet 245 has slits 245e extending in the longitudinal direction formed at positions spaced right and left apart from the skin contacting portion 21. The slit 245e passes through the surface sheet 245. As shown in Fig. 6, a front end of the slit 245e is positioned slightly ahead of a front end of a first region 31, and a rear end of the slit 245e is positioned slightly behind a rear end of the first region 31.

In the absorbent article 201, the slits 245e are formed in both side parts of the surface sheet 245 covering the skin contacting portion 21. Therefore, the surface sheet 245 does not restrict the degree of freedom of the skin contacting portion 21, so that a state where the skin contacting portion 21 adheres to a urine excretory part through the surface sheet 245 is easily maintained. When a large amount of urine is supplied to the surface sheet 245, the urine is supplied to an absorbent body 13 after passing through the slits 245e, so that the urine does not easily remain on the surface sheet 245. Moreover, the skin contacting portion 21 is covered with the surface sheet 245. Therefore, when an elderly woman, for example, employs the absorbent article 201, its surface on the liquid receiving side can be seen as being smooth, so that she does not readily feel uncomfortable and awkward at the time of wearing.

Furthermore, through portions such as the slits 245e formed in the surface sheet 245 are not limited to the slits continuously extending in the longitudinal direction, as described above. Short slits each having a predetermined length in the longitudinal direction may be intermittently spaced apart in the longitudinal direction. Alternatively, through portions such as rectangular, elliptical, and circular through portions may be spaced apart in the longitudinal direction.

It should be noted that the absorbent article according to the present invention is not used only as a urine collection pad, but is usable as a sanitary napkin. In the case of the sanitary napkin, the skin contacting portion 21 can adhere to the excretory part. When a large amount of liquid is supplied, the liquid is stored in the cavities 26 and 27 so that menstrual blood or the like can be absorbed by the absorbent body 13.

While preferred embodiments of the present invention have been described and illustrated above, it is to be understood that they are exemplary of the invention and are not to be considered to be limiting. Additions, omissions, substitutions, and other modifications can be made thereto without departing from the spirit or scope of the present invention. Accordingly, the invention is not to be considered to be limited by the foregoing description and is only limited by the scope of the appended claims.

## Claims

1. An absorbent article having an absorbent body, a liquid-permeable sheet provided on a liquid receiving side of the absorbent body, and a back sheet provided on the side opposite to wearing clothing side of the absorbent body and in such a vertically-long shape that a size thereof in a longitudinal direction is greater than a size thereof in a transverse direction, wherein
the liquid-permeable sheet has a skin contacting portion extending along a center line in the longitudinal direction, side fixing portions fixed to a surface on the liquid receiving side of the absorbent body on both sides of the skin contacting portion, respectively, and a pair of raised portions connecting the skin contacting portion and the side fixing portions,
the skin contacting portion is provided with at least one elastic expansion/contraction portion, and is spaced apart from the surface of the absorbent body with an elastic contractive force in the longitudinal direction of the elastic expansion/contraction portion, and
at least one cavity surrounded by the skin contacting portion and the raised portions, and extending in the longitudinal direction is formed on the absorbent body.

2. The absorbent article according to claim 1, wherein the at least one elastic expansion/contraction portion comprises a plurality of elastic expansion/contraction portions spaced apart in the transverse direction, and the skin contacting portion is spaced a predetermined width apart from the surface of the absorbent body.

3. The absorbent article according to claim 1 or 2, wherein the at least one elastic expansion/contraction portion has a first elastic member, extending in the longitudinal direction, joined to the liquid-permeable sheet.

4. The absorbent article according to any one of claims 1 to 3, wherein the liquid-permeable sheet has deflection allowances in portions forming the raised portions, respectively, between the side fixing portions and the skin contacting portion.

5. The absorbent article according to any one of claims 1 to 4, wherein
the skin contacting portion has a first region and a second region positioned behind the first region, the height thereof raised from the surface of the absorbent body in the first region being greater than that in the second region, and
the at least one cavity comprises cavities respectively formed in both the first region and the second region.

6. The absorbent article according to any one of claims 1 to 4, wherein
the skin contacting portion has a first region and a second region disposed behind the first region, the height thereof raised from the surface of the absorbent body in the first region being greater than that in the second region,
the at least one cavity comprises cavities formed in both the first region and the second region, respectively, and
the at least one elastic expansion/contraction portion is provided in the first region, while not being provided in the second region.

7. The absorbent article according to any one of claims 1 to 4, wherein
the skin contacting portion has a first region and a second region disposed behind the first region, the height thereof raised from the surface of the absorbent body in the first region being greater than that in the second region,
the at least one cavity comprises cavities formed in both the first region and the second region, respectively, and
the distance in the width direction between a base end of one of the raised portions and a base end of the other raised portion in the second region is greater than that in the second region.

8. The absorbent article according to any one of claims 1 to 7, wherein the absorbent body has leakage preventing walls each formed of a side sheet and a second elastic member, and extending in the longitudinal direction in both side parts on a surface thereof on the liquid receiving side, the leakage preventing walls being curved in a state where the surface on the liquid receiving side of the absorbent body is recessed with an elastic contractive force of the second elastic member.

9. The absorbent article according to any one of claims 1 to 8, wherein the absorbent body has absorbing core compression portions formed by compressing an absorbing core in the absorbent body, and the absorbing core compression portions are spaced right and left apart from the center line in the longitudinal direction and are formed continuously in the longitudinal direction with a predetermined width.

10. The absorbent article according to any one of claims 1 to 9, wherein a liquid-permeable surface sheet covers both the skin contacting portion and the side fixing portions.

11. The absorbent article according to claim 10, wherein the surface sheet is stretchable.

12. The absorbent article according to claim 10 or 11, wherein the surface sheet has through portions formed in both side parts of the skin contacting portion.
